# EUROPEAN PATENT SPECIFICATION

(11) **EP 3 398 573 B1**
(45) Date of publication and mention of the grant of the patent: **14.04.2021**
(21) Application number: 17169713.9
(22) Date of filing: 05.05.2017
(51) Int. Cl.: A61F 13/06, A61F 13/08

(54) **COMPRESSION BANDAGE**
KOMPRESSIONSBANDAGE
BANDAGE DE COMPRESSION

(43) Date of publication of application: 07.11.2018
(73) Proprietor: 3M Innovative Properties Company, Saint Paul, MN 55133-3427 (US)
(72) Inventor: Hitschmann, Guido, 41470 Neuss (DE)
(74) Representative: Gabriel, Kiroubagaranne

(56) References cited:
- WO-A1-2004/058122
- JP-A- H10 305 052
- US-B1- 6 545 193

## Description

The invention relates to elastic compression bandages and to compression stockings for compression therapy of venous leg ulcer on human calves. It relates in particular to such compression bandages which are designed to be wound around the calf in multiple turns.

Compression bandages are known for use in the treatment of edema, as well as venous and lymphatic disorders, e.g., of the lower limbs. Areas where compression bandages are considered particularly useful are the management and treatment of chronic wounds, such as venous leg ulcers. Compression bandages are one of the common compression systems used for compression therapy. Due to their elasticity, elastic compression bandages can conform well to the contour of a patient's calf. Compression therapy of venous leg ulcer involves providing high pressure on the veins in a patient's calf when the patient is standing. Only a sufficiently high pressure supports the so-called muscle pump adequately to support healing. Lower pressure levels are not considered therapeutic pressure levels.

Generally, unstretched, loose portions of the bandage do not provide therapeutic pressure. Compression bandages should, however, conform to the contours of a calf sufficiently to provide therapeutic compression also to those portions of the calf that have a smaller diameter. For this reason, compression bandages are generally elastically stretchable in length direction of the bandage, so that they can be easily stretched, with comparatively little force, just before application to the calf. Where the same turn of the bandage covers larger-diameter portions and smaller-diameter portions of the calf, the bandage is stretched sufficiently before application so that even the smaller-diameter portions are still covered by a stretched portion of the bandage and don't hang loose or form loose pockets. "Soft", easily-stretchable bandages have a lower modulus of elasticity.

On the other hand, an effective compression bandage is supposed to provide a substantial resistance against even small increases in calf diameter, which occur for instance when the patient moves from a resting position into a standing position. Bandages that provide such a resistance are often called "inelastic" and will be referred to as "stiff" in this disclosure. Stiff bandages have a higher modulus of elasticity than soft bandages at the elongation at which they are applied. Stiff bandages are able to provide more effective compression through a low resting pressure and a high standing or walking pressure. In other words, stiff bandages result in a higher Static Stiffness Index, the difference between standing pressure and resting pressure being greater than about 10 mm Hg. The use of an inelastic, "stiff" compression bandage is especially advantageous for patients with venous insufficiency, since the low resting pressure provides comfort when the patient is recumbent while also preventing expansion of muscle diameter while the patient is standing or walking, thereby increasing venous and lymphatic return when the muscles of the leg contract. The International Patent Application WO 2013/048755 A1 describes such inelastic, stiff bandages.

The resulting resting pressure of soft (e.g. long stretch) bandages can be controlled well, but the sub-bandage standing pressure of such systems is not much higher than the resting pressure, resulting in a less effective compression. On the other hand, stiff (inelastic, e.g. short-stretch) bandages are designed to provide a standing pressure which is much higher than the resting pressure, resulting in higher compression efficacy. Traditional bandages were designed to balance stiffness versus softness, often compromising both. United States Patent US 6 545 193 B1 discloses an elongated elastic bandage which includes a plurality of hook fastener strips on a first side and a plurality of loop fastener strips on a second side. In use, the described hook and loop fastener strips form a criss-cross configuration when the elastic bandage encircles a portion of a user's body.

The present disclosure attempts to address this problem. It provides an elongate elastic compression bandage for application around a human calf in circumferential turns for applying a sufficient therapeutic pressure on the calf for treatment of venous leg ulcer, the bandage having a first major surface and an opposed second major surface, wherein the long extension of the bandage, when arranged flat, defines a length direction, and wherein the bandage comprises, arranged in its length direction, a) a first engagement zone, arranged on the first major surface and comprising mechanical engagement means, b) a second engagement zone, arranged on the first major surface and comprising mechanical engagement means, c) a soft section, arranged, in length direction of the bandage, between the first engagement zone and the second engagement zone, and having a reduced modulus of elasticity in length direction, d) a stiff section having an elevated modulus of elasticity in length direction, higher than the reduced modulus, and having a length greater than the length of the soft section, where a length of a section is determined by the longest extension of the section in the length direction of the unexpanded bandage, and comprising a third engagement zone arranged on the second major surface, comprising mechanical engagement means, for engagement with the first engagement zone and with the second engagement zone, wherein the sections and the engagement zones are arranged such, that, when applying the bandage around the calf in circumferential turns, a first portion of the third engagement zone can engage, in a subsequent turn, with the first engagement zone, and that a second portion of the third engagement zone can engage, with the second engagement zone, so that the stiff section bridges the soft section.

In the first circumferential turn (and potentially further turns, depending on the position of the stiff section in length direction) of the bandage around the calf the soft section is not yet bridged and remains easily stretchable. The bandage as a whole is therefore softer than the stiff section alone, and can conform to parts of the calf having smaller diameters without forming loose portions

In the second circumferential turn (or a later turn, depending on the position of the stiff section in length direction), following engagement of the engagement zones, the soft section is bridged by the stiff section. The lower elastic modulus properties of the soft section are overridden by the higher elastic modulus of the stiff section, whereby the bandage as a whole becomes stiffer and presents a higher resistance to increases in the diameter of the calf.

The engagement of the third engagement zone of the stiff section with the respective first and second engagement zones of the two stiff sections adjacent to the soft section, and the resulting bridging of the soft section of the bandage by the stiff section, changes the properties of the bandage as a whole towards a higher stiffness than it had before the engagement. This is desirable for an effective compression therapy of venous leg ulcer.

The elongate elastic compression bandage according to the present disclosure is as described in the appended claims.

A bandage according to the present disclosure has a common geometry: It is elongate, and when arranged flat, its long extension defines a length direction of the bandage. The bandage is long enough for being wound around a typical human calf in at least two full circumferential turns. Circumferential turns are turns following grossly the circumference of the calf. In a direction (the "width direction") orthogonal to the length direction, the bandage extends between a first side edge and a second side edge. The side edges may be parallel to each other. Thickness directions of the bandage are directions orthogonal to both length directions and width directions. Bandages according to this disclosure may have a length, when unexpanded, of between 15 centimetres (cm) and 500 cm, preferably of between 70 cm and 300 cm. Bandages according to this disclosure may have a width, when unexpanded, of between 5 cm and 20 cm, preferably of between 7 cm and 15 cm.

A section of the bandage refers to a longitudinal portion of the bandage, e.g. as a result of subdividing the bandage in its length direction. A section may extend from the first side edge to the second side edge.

The bandage may extend in width directions between a first side edge and a second side edge. The second side edge may be parallel to the first side edge.

The stiff section and the soft section may extend from the first side edge to the second side edge. In bandages according to this disclosure which have a plurality of stiff sections, each stiff section may extend from the first side edge to the second side edge. In bandages according to this disclosure which have a plurality of soft sections, each soft section may extend from the first side edge to the second side edge.

A bandage comprising rectangular-shaped sections may be particularly cost-effective to manufacture. Therefore, each stiffening section may have a rectangular shape and may extend from the first side edge to the second side edge. Each soft section may have a rectangular shape and may extend from the first side edge to the second side edge.

Alternatively, each stiffening section may have a trapezoid shape and may extend from the first side edge to the second side edge. Each soft section may have a trapezoid shape and may extend from the first side edge to the second side edge.

Generally, any section of the bandage may have a length. In the context of the present disclosure, the length of a section refers to the greatest extension of the section in length direction of the bandage, when the bandage is unexpanded and arranged flat.

In the present disclosure, the modulus of elasticity is referred to as a measure for the resistive force of a section of the bandage, or of the bandage as a whole, against (further) expansion in length direction. For elastic fabrics, often used for compression bandages, the modulus is expressed in Newton per cm of (additional) expansion, per cm width of the tested specimen and per cm thickness of the specimen. In the present disclosure, however, the thickness of the specimen is not taken into account and the "modulus of elasticity" is independent on the thickness of the specimen. Herein, the modulus of elasticity refers to the force per percent elongation per width, i.e. the modulus of elasticity in its traditional meaning, multiplied by the thickness of the specimen.

Generally, the stiff section of a bandage according to the present disclosure must have a modulus of elasticity which is higher than the modulus of elasticity of the soft section. The modulus of elasticity of the (or a) stiff section may be, for example, more than 10% higher, more than 30% higher, or even more than 50% higher than the modulus of elasticity of the (or a) soft section. This ensures that the stiffening effect of the bridging on the bandage is obtained. The absolute values of the modulus of elasticity of the stiff section and of the soft section are less relevant for effective bridging. A material having a certain modulus of elasticity may be used to form a stiff section in one type of bandage, while the same material may be used to form a soft section in another type of bandage.

Materials for use in a stiff section of compression bandages according to the present disclosure ("stiff materials") have an elevated modulus of elasticity, i.e. a modulus of elasticity of greater than 0.03 N/cm/%, measured at an elongation of 5% over the unstretched length, independent of the thickness of the material. "N/cm/%" refers to the elastic force in Newton, divided by the width of the material in centimetres, and divided by the percent additional elongation over the 5% elongation. Percent elongation is determined by dividing the elongated length by the unstretched length and multiplying with 100. Preferable stiff materials have a modulus of elasticity of greater than 0.30 N/cm/%, measured at an elongation of 5% over the unstretched length. A typical modulus of elasticity of a stiff material is 0.40 N/cm/%, measured at an elongation of 5% over the unstretched length.

Materials for use in a soft section of compression bandages according to the present disclosure ("soft materials") have a reduced modulus of elasticity, i.e. a modulus of elasticity of less than 0.10 N/cm/%, measured at an elongation of 5% over the unstretched length. Modulus of elasticity can be measured on many commercially available tensile elongation testing machines. The modulus of elasticity of a section of a bandage may be determined, for example, by measuring the modulus of elasticity of a 5 cm wide specimen of the material, of which the section is made, in stretch direction, i.e. in length direction of the bandage on a tensile elongation testing machine at room temperature (22°C) at an elongation of 5% over the unstretched length.

A modulus of elasticity in length direction may be different at different elongations of the bandage. In the context of the present disclosure, the modulus of elasticity normally refers to the modulus at the elongation at which the bandage is wrapped around the patient's calf. In the context of the present disclosure, a modulus of elasticity is measured at an elongation of 5% over the unstretched length.

Once the stiff section bridges the soft section, its elasticity determines the stiffness of the bandage in the region of the bandage in which the stiff section is engaged. For effective therapeutic compression, the modulus of elasticity is advantageously comparatively high. The stiff section has a modulus of elasticity in length direction of more than 0.03 N/cm/%, measured at an elongation of 5% over the unstretched length. Particularly, the stiff section may have a modulus of elasticity in length direction of more than 0.10 N/cm/%, measured at an elongation of 5% over the unstretched length. The stiff section may be rendered rigid by a very high modulus of elasticity. In bandages according to this disclosure which have a plurality of stiff sections, each stiff section has a modulus of elasticity in length direction of more than 0.03 N/cm/%, measured at an elongation of 5% over the unstretched length.

In order for the bandage to conform to the contours of the calf, the soft section advantageously has a relatively low modulus of elasticity in length direction of the bandage. The soft section has a modulus of elasticity in length direction of less than 0.10 N/cm/%, preferably less than 0.05 N/cm/%, measured at an elongation of 5% over the unstretched length. In bandages according to this disclosure which have a plurality of soft sections, each soft section has a modulus of elasticity in length direction of less than 0.10 N/cm/%, measured at an elongation of 5% over the unstretched length. Preferable soft materials have a modulus of elasticity of less than 0.05 N/cm/%, measured at an elongation of 5% over the unstretched length. A typical modulus of elasticity of a soft material is 0.04 N/cm/%, measured at an elongation of 5% over the unstretched length.

The sections of a bandage according to the present disclosure may be elastic. In particular they may be elastic in length direction of the bandage. Generally, an elastic section, when expanded or stretched in length direction by a force typically used when applying compression bandages, strives to return to its original, unexpanded length. An expanded elastic section, and an expanded elastic bandage, when applied in an expanded state around the calf, strives to return to its original unexpanded length, and the resulting contraction force exerts therapeutic compression on the calf.

Any of the sections may be made of, or comprise, an elastic material, e.g. an elastic fabric. Such fabrics are known, and fabrics having different modulus of elasticity, such as Spandex, Lycra or elasthane-containing fabrics, can be obtained commercially, e.g from Fabric.com, Invista, Taekwang, Hyosung, Indorama Corporation, Asahi Kasei or Toyobo under brand names such as Lycra, Elaspan, Acepora, Creora, Inviya, ROICA, Dorlastan, Linel or ESPA.

The soft section, any soft section or all soft sections may have a reduced modulus of elasticity. The stiff section, any stiff section or all stiff sections may have an elevated modulus of elasticity. Any section may extend from the first side edge of the bandage to the second side edge for at least a portion of its length.

An engagement zone on a section of the bandage refers to the portion, or collectively to those portions, of the section that comprise(s) mechanical engagement means. An engagement zone can thus consist of one or two or more portions that each comprise mechanical engagement means. An engagement zone may comprise different types of engagement means. A portion of an engagement zone may comprise different engagement means. Due to the presence of the mechanical engagement means, an engagement zone is adapted to mechanically engage with another engagement zone. The expression "engaging" or "engagement", in the context of this disclosure, refers to establishing a mechanical connection between two elements that prevents relative movement of the engaged elements in length directions of the bandage. In a bandage according to the present disclosure, a first portion of the third engagement zone can engage with the first engagement zone, and a second portion of the third engagement zone can engage with the second engagement zone.

In the context of the present disclosure, mechanical engagement means may be, for example, an adhesive-carrying surface. Such a surface may mechanically engage, via the adhesive, with another surface, adhesive-coated or not, by bringing the surfaces into mechanical contact with each other. The adhesive provides a mechanical engagement between the surfaces which prevents relative movement of the surfaces, e.g. in a direction parallel to the surfaces.

Alternatively, or in addition, mechanical engagement may be obtained by magnetic means. The mechanical engagement means may thus comprise a magnet. Using magnetic means, a surface of a magnetized element can contact a surface of an opposed magnetized element. The magnets, properly oriented, will attract each other, so that their surfaces contact each other. The magnetic force may increase friction between the contacting surfaces, which in turn prevents relative movement of the surfaces in a direction parallel to the surfaces.

In certain embodiments, an engagement means comprises a first component of a two-component connection system, such as, for example, a two-component mechanical fastening system. The first component can engage with a second component of the two-component connection system to build a mechanical connection and prevent relative movement of the engaged elements in length directions of the bandage. Examples of two-component connection systems are hook-and-loop type fastening systems, mechanical fastener type connection systems such as those comprising surfaces carrying mushroom-shaped microstructures that can engage with an opposed surface also carrying such mushroom-shaped microstructures.

Hook-and-loop type engagement can be achieved by one engagement zone having a surface covered with engagement means in the form of small hooks, and by the corresponding opposite engagement zone having a surface covered with engagement means in the form of many small loops of filaments. The hooks, or a surface provided with hooks, is a component of a two-component connection system. The loops, or a surface provided with loops, is a component of a two-component connection system. When the zones are being brought in contact, the loops are entangled with the hooks and thereby engage the engagement zones. As a result, relative movement between the surfaces of the engagement zones, in particular in directions parallel to the surfaces, and/or, if suitably arranged, in the length direction of the bandage, is prevented. An example of a hook-and-loop-type engagement is the well-known Velcro(R) fastening system. Materials usable as engagement means in bandages, stockings or wraps according to the present disclosure are "Loop 001", available from Velcro or "Hook 088", also from Velcro. These materials are stiff, so that they can be used to form a stiff section in bandages according to the present disclosure. Alternatively they can be affixed to a surface of a stiff material forming a stiff section. Alternative hook materials are available form Gottlieb Binder GmbH & Co. KG of Holzerlingen, Germany.

An engagement zone may thus comprise engagement means in the form of a fabric, or a surface of a fabric, e.g. of a non-woven fabric, that is suitable to engage with a hook-type surface or with a mushroom-structured surface. An engagement zone may comprise engagement means in the form of a hook-type surface that is adapted to engage with a loop-type surface. An engagement zone may comprise engagement means in the form of a loop-type surface that is adapted to engage with a hook-type surface. In certain embodiments, the first and/or the second engagement zone comprises engagement means in the form of a plurality of hooks, and the third engagement zone comprises engagement means in the form of a plurality of loops, shaped suitably to be mechanically engaged with hooks identical to the hooks of the first and/or the second engagement zone. In certain other embodiments, the first and/or the second engagement zone comprises engagement means in the form of a plurality of loops, and the third engagement zone comprises engagement means in the form of a plurality of hooks, shaped suitably to be mechanically engaged with loops identical to the loops of the first and/or the second engagement zone.

So-called mechanical fastening systems are known fastening systems. They provide engagement between two opposed engagement zones, the surfaces of which are covered with a large number of mushroom-shaped elements. When bringing the zones into contact with each other under a certain moderate pressure, the "roofs" of the mushroom elements catch each other and prevent relative movement, in particular in directions parallel to the surfaces and/or in the length direction of the bandage, if suitably arranged. Engagement means in the form of mechanical fastening systems may be used in a compression bandage according to the present disclosure to engage the third engagement zone with the first engagement zone and the second engagement zone. In such embodiments of a bandage, the first and/or the second engagement zone comprises engagement means in the form of a plurality of mushroom-shaped fastening elements, and the third engagement zone, or at least a portion of it, comprises engagement means in the form of a plurality of mushroom-shaped fastening elements, adapted to be mechanically engaged with mushroom-shaped fastening elements identical to the mushroom-shaped fastening elements of the first and/or second engagement zone.

Alternatively or in addition, mechanical engagement can be achieved via one or more friction-enhancing layers. Where an engagement zone comprises engagement means in the form of an exposed friction-enhancing layer, e.g. a layer of a tacky adhesive, this engagement zone may be brought in contact with an opposed engagement zone which has engagement means in the form of a "landing zone" for contacting the opposed friction-enhancing layer. Under moderate pressure, the friction-enhancing layer on one engagement zone will stick to the landing zone on the corresponding opposite engagement zone and prevent relative movement in directions parallel to the contact surfaces of the engagement zones and/or in the length direction of the bandage.

Hence, in certain embodiments of a bandage according to this disclosure, the first engagement zone and/or the second engagement zone comprises engagement means in the form of an exposed friction-enhancing layer, adapted to be mechanically engaged with an exposed surface identical to an exposed surface of the third engagement zone. In certain embodiments, the third engagement zone comprises engagement means in the form of an exposed friction-enhancing layer, adapted to be mechanically engaged with an exposed surface identical to an exposed surface of the first engagement zone or the second engagement zone.

For a stronger engagement effect, both opposed engagement zones may comprise engagement means in the form of a respective friction-enhancing layer. Hence, in certain embodiments of a bandage according to this disclosure, the first and/or the second engagement zone comprises engagement means in the form of an exposed friction-enhancing layer, and the third engagement zone comprises engagement means in the form of an exposed friction-enhancing layer, adapted to be mechanically engaged with a friction-enhancing layer identical to the friction-enhancing layer of the first and/or second engagement zone.

Alternatively or in addition, mechanical engagement can be achieved via one or more exposed adhesive layers. Where an engagement zone has engagement means in the form of an exposed adhesive layer, e.g. a layer of a tacky adhesive, this engagement zone may be brought in contact with an opposed engagement zone which has engagement means in the form of a "landing zone" for contacting the opposed adhesive layer. Under moderate pressure, the adhesive layer on one engagement zone will stick to the landing zone on the corresponding opposite engagement zone and prevent relative movement in directions parallel to the contact surfaces of the engagement zones and/or in the length direction of the bandage. Hence, in certain embodiments of a bandage according to this disclosure, the first and/or the second engagement zone comprises engagement means in the form of an exposed adhesive layer, and the third engagement zone comprises engagement means in the form of an exposed landing zone, adapted to be mechanically engaged with an adhesive layer identical to the adhesive layer of the first and/or the second engagement zone.

For a stronger engagement effect, both opposed engagement zones may comprise engagement means in the form of a respective adhesive layer. Hence, in certain embodiments of a bandage according to this disclosure, the first and/or the second engagement zone comprises engagement means in the form of an exposed adhesive layer, and the third engagement zone comprises engagement means in the form of an exposed adhesive layer, adapted to be mechanically engaged with an adhesive layer identical to the adhesive layer of the first and/or second engagement zone.

An engagement zone may be arranged on a major surface of the section in which it is comprised. Particularly, the first engagement zone may be arranged on a major surface of the first compression section. Preferably it is arranged on the major surface of the first compression zone that is oriented radially outward when the bandage is applied around the calf.

The second engagement zone may be arranged on a major surface of the second compression section. Preferably it is arranged on the major surface of the second compression zone that is oriented radially outward when the bandage is applied around the calf.

The third engagement zone may be arranged on a major surface of the stiff section. Preferably it is arranged on the major surface of the stiff zone that is oriented radially inward when the bandage is applied around the calf.

An engagement zone may cover a certain area of a major surface of the section in which it is comprised. It may, for example, cover an area of between 2 square centimetres (cm²) and 200 cm². For ease of application it may be preferable, however, that an engagement zone covers an area of between 10 cm² and 100 cm².

A material of a section (e.g. first compression section, second compression section or stiff section) may be chosen such that one of its major surfaces is suitable for mechanical engagement by an engagement zone. In such cases, an entire major surface of a section may form an engagement zone.

The soft section of a bandage according to the present disclosure has a reduced modulus of elasticity, as defined above, in length direction of the bandage. The soft section may extend from the first side edge of the bandage to the second side edge. It may extend from the first side edge of the bandage to the second side edge for at least a portion of its length.

For a given length of the bandage, the length of the soft section impacts the stiffness of the bandage. If the soft section occupies a larger percentage of the length of the bandage, the bandage will conform better to the contours of the patient's calf. However, a shorter soft section may make the bandage stiffer and thus allow for a higher degree of therapeutic compression. The length of a soft section should be smaller than the circumference of the calf. The soft section may have a length of between 1 cm and 20 cm, when the bandage is unexpanded and arranged flat. Preferably, the soft section may have a length of between 3 cm and 10 cm, when the bandage is unexpanded and arranged flat. Preferably, the length of the soft sections and the length of the stiff sections are chosen such that between three and six soft sections are present for each turn of the bandage around a typical human calf.

Where a bandage according to this disclosure has further soft sections, the length of a plurality of soft sections may be equal. In that case, the length of each soft section may be, for example, between 1 cm and 20 cm.

A suitable material to form a soft section in bandages according to the present disclosure is "Coban LF", available from 3M Company, St. Paul, Minnesota, U.S.A.

The stiff section of a bandage according to the present disclosure has an elevated modulus of elasticity, as defined above, in length direction of the bandage. The stiff section may extend from the first side edge of the bandage to the second side edge. It may extend from the first side edge of the bandage to the second side edge for at least a portion of its length.

For a given length of the bandage, the length of the stiff section impacts the stiffness of the bandage, before the bandage is applied around the calf. If the stiff section occupies a larger percentage of the length of the bandage, the bandage is generally stiffer and may thus allow for a higher degree of therapeutic compression. The stiff section, or each stiff section, may have a length of between 1 cm and 20 cm. Preferably, the stiff section, or each stiff section, may have a length of between 3 cm and 10 cm.

Where a bandage according to this disclosure has further stiff sections, the length of a plurality of stiff sections may be equal. In that case, the length of each stiff section may be, for example, between 1 cm and 20 cm.

The stiff section may have, in length direction of the bandage, a first end portion and an opposed second end portion. The first end portion may comprise the first portion of the third engagement zone. The second end portion may comprise the second portion of the third engagement zone.

In other words, the first end portion of the stiff section may be located at one longitudinal end of the stiff section, and the second end portion maybe located at an opposite longitudinal end of the stiff section. The first portion of the third engagement zone of the stiff section may be arranged at one longitudinal end, e.g. with an edge of the third engagement zone located within 2 cm or 5 cm from the one longitudinal end of the stiff section. The second portion of the third engagement zone of the stiff section may be arranged at the opposite longitudinal end, e.g. with an edge of the second portion of the third engagement zone located within 2 cm or within 5 cm from the opposite longitudinal end of the stiff section.

Stiff elastic fabrics are often quite dense and do not allow adequate amounts of air to pass through. This may cause skin irritation underneath a stiff section. In order to avoid such irritations, the stiff section may comprise apertures for facilitating transmission of air between the lower major surface and the upper major surface. Where the compression bandage comprises a plurality of stiff section, at least one stiff section may comprise apertures for facilitating transmission of air between the lower major surface and the upper major surface.

A compression bandage according to the present disclosure is suitable for being wound around a human calf in multiple turns. This implies that the bandage is flexible, i.e. it can be deformed such as to follow the contours of a calf. Flexibility is considered to independent from elasticity, in the context of the present disclosure. In order for the bandage to be flexible, all sections of the bandage are preferably flexible. The first compression section and/or the second compression section may be flexible. The soft section may be flexible. The stiff section may be flexible.

However, alternatively, one or more sections may be inflexible, i.e. rigid. In particular, the stiff section may be rigid. If a stiff section is rigid, its modulus of elasticity in length direction is very high, and may have a value that is typical of solid, non-elastic bodies.

When the engagement zones of the stiff section are engaged with the respective engagement zones of the first and the second compression sections, the stiff section bridges the soft section. When bridging, the stiff section may extend parallel to the soft section, i.e. the major surfaces of the stiff section and of the soft section may be parallel to each other, both conforming around the patient's calf. The stiff section has a length, as defined herein, which is greater than the length of the soft section, when the bandage is unexpanded and arranged flat.

Certain bandages according to this disclosure will be applied around the calf in turns, where a subsequent turn overlaps with the previous turn by about half the width of the bandage. In these bandages, the stiff section will bridge the soft section underneath it, although it may only overlap the soft section by half its width. Bridging is considered to be present from 100% overlap in width down to about 10% overlap in width between stiff section and underlying soft section.

Bridging of the soft zone by the stiff zone results in the bandage being stiffer in the area of the soft zone, because the stiff zone has an elevated modulus of elasticity, compared to the modulus of the soft zone. When the diameter of the calf increases slightly, the bandage in the area of the soft zone can only be expanded against the (higher) resistance of the stiff zone, whereas before bridging it could be expanded against the (lower) resistance of the soft zone.

The soft section is adapted to be expanded before application of the bandage around the calf. In order for the third engagement zone on the stiff section to be more easily engageable with the first and with the second engagement zones, the first portion and the second portion are preferably spaced from each other by a distance in length direction which is greater than the length of the soft section, when the bandage is unexpanded and arranged flat. Hence the first portion and the second portion of the third engagement zone may be spaced from each other in length direction of the bandage by a longitudinal distance greater than the length of the soft section, when the bandage is unexpanded and arranged flat.

In order for a bandage according to the present disclosure to be usable for treatment of calves of many different circumferences, the bandage advantageously comprises more than one soft section, and more than one stiff section. A bandage according to the present disclosure may therefore comprise
a) a plurality of soft sections, each soft section having a reduced modulus of elasticity in length direction, and
b) a plurality of stiff sections, each having an elevated modulus of elasticity in length direction, the plurality of stiff sections being arranged alternating with the plurality of soft sections in length direction of the bandage.
Each stiff section of the plurality of stiff sections comprises, on the first major surface, a first engagement zone and a second engagement zone, and, on the second major surface, a third engagement zone, the third engagement zone being arranged such that a first portion of the third engagement zone can engage with the first engagement zone of a first other stiff section of the plurality of stiff sections, and such that a second portion of the third engagement zone can engage with the second engagement zone of a second other stiff section of the plurality of stiff sections, so that the stiff section bridges the soft section between the first other and the second other stiff section.

This bandage may consist of a sequence of "soft section - stiff section - soft section - stiff section etc", with no other section between a soft zone and an adjacent stiff zone. Such bandages may be particularly cost-effective to manufacture.

The arrangement of the first engagement zone and the second engagement zone on the first major surface of each stiff section facilitates their engagement with a third or fourth engagement zone of another stiff section, that may be positioned over the said stiff section in a subsequent turn of the bandage, when the bandage is wound around the calf.

The arrangement of the third engagement zone on the second major surface of each stiff section facilitates its engagement with a first or second engagement zone of another stiff section, that may have been positioned underneath the said stiff section in a previous turn of the bandage, when the bandage is wound around the calf.

In a bandage having a plurality of stiff sections and a plurality of soft sections, the area occupied by the stiff sections has an impact on the overall stiffness of the entire bandage. "Area" refers to the geometric surface area which is covered by the bandage when laid out flat in an unstretched state. When using typical, commercially available elastically stretchable materials of elevated modulus of elasticity for the stiff sections, it has been found advantageous if the stiff sections occupy more than half of the geometric bandage area, when the bandage is unexpanded and arranged flat. Such bandages have a suitable overall stiffness for efficient compression therapy of venous leg ulcer. Hence, in certain embodiments of the bandage according to the present disclosure, the bandage, when unexpanded and arranged flat, covers a geometric bandage area, and wherein the plurality of stiff sections occupy at least 50% of the geometric bandage area. In particular, the plurality of stiff sections may occupy at least 70% of the geometric bandage area.

Where a soft zone is not bridged by a subsequent stiff zone, the bandage remains easily stretchable in the area of the soft zone. In that area it may not provide sufficient therapeutic pressure on the calf. It is thus desirable to avoid such "unbridged" soft zones.

Since many bandages according to the present disclosure may be applied to a calf with an overlap of about 50% in width between a lower turn and a subsequent upper turn, the right-hand side (when looking in length direction of the bandage towards its not-yet-applied end) of a lower turn will be covered by a left-hand side of a subsequent upper turn or vice versa. Unbridged soft zones can thus be reduced or avoided if the right-hand side of each stiff section is provided with an engagement zone extending linearly in a first diagonal direction (such as forming an angle of +45% with the length direction of the bandage in the plane of the bandage when arranged flat), and an engagement zone extending linearly in a second diagonal direction (such as forming an angle of -45% with the length direction of the bandage in the plane of the bandage when arranged flat). When a first turn of the bandage is covered by a subsequent turn with 50% overlap in width, the engagement zone on the upper major surface of the stiff section in the first turn and the engagement zone on the lower major surface of the stiff section in the subsequent turn form an "X" pattern with crossing diagonal lines. In this geometry, engagement and bridging is very likely to happen. A "Chevron" shape combines diagonals in two different directions, arranged side by side in width direction. Engagement zones in such a chevron shape therefore appear advantageous.

Hence, in the bandage according to the present disclosure, the third engagement zone of at least two subsequent stiff sections have a congruent "chevron" shape and point in the same length direction of the bandage. The at least two chevron shapes may be spaced from each other in length direction by one or more respective chevron-shaped soft sections.

Where the stiff sections have a chevron shape extending from one side edge to the opposed side edge, and the soft sections have a chevron shape extending from one side edge to the opposed side edge, at least one stiff section must have a sufficient length to bridge at least one soft section. In certain embodiments, the length of each chevron-shaped soft section may be smaller than the length of any one of the chevron-shaped stiff sections. In embodiments in which at least two subsequent stiff sections each have congruent chevron shapes, oriented parallel to each other and pointing in the same length direction of the bandage, spaced from each other in length direction by one or more respective chevron-shaped soft sections, the length of each of the chevron-shaped soft sections may be less than 80% of the smallest length of each of the at least two chevron-shaped stiff sections.

Compression bandages as described herein may be for use in a single-layer compression bandage system or alternatively in a two-layer compression bandage system, in which an outer bandage is wrapped around an inner bandage. They may be used, for example, as an outer bandage, applied over an inner bandage which forms a cushioning layer or a comfort layer. In a yet alternative use, a bandage as described herein may be usable as an inner layer in a two-layer compression system, in which an elastic stocking forms the outer layer. Such a stocking may improve the visual appearance of the compression system when worn, and/or may prevent the inner compression bandage from loosening or slipping on the calf.

The present disclosure will now be described in more detail with reference to the following Figures exemplifying particular embodiments of the disclosure.
- Fig. 1: Perspective view of a first compression bandage not according to the present disclosure;
- Fig. 2: Side view of the first compression bandage, applied in two turns;
- Fig. 3: Top view of a second compression bandage according to the present disclosure;
- Fig. 4: Bottom view of the second compression bandage;
- Fig. 5: Top view of the second compression bandage, applied in two layers;
- Fig. 6: Plan view of a compression stocking not according to the present disclosure;
- Fig. 7: Plan view of a stiffening wrap not according to the present disclosure;
- Fig. 8: Perspective view of the compression stocking in use, with the stiffening wrap applied over it; and
- Fig. 9: Sectional view of a flexible material for use in a bandage according to the present disclosure.

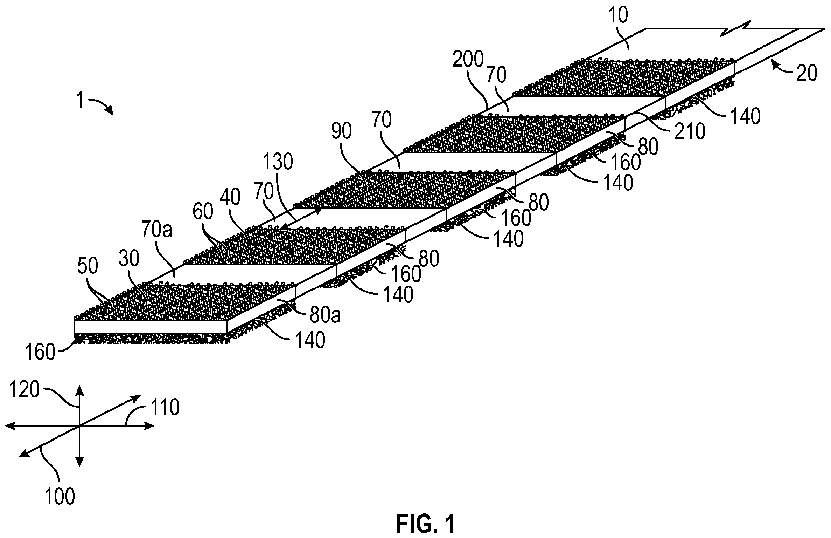

The perspective view of **Figure 1** is an illustration of a first compression bandage not according to the present disclosure. The compression bandage 1 is shown arranged flat and unexpanded. It has a first major surface 10 and an opposed second major surface 20. The long extension of the bandage 1 defines a length direction of the bandage 1, indicated by arrow 100. The width of the bandage 1 defines width directions 110, and the thickness of the bandage defines thickness directions 120.

Arranged sequentially in its length direction 100, the bandage 1 comprises, in an alternating sequence, a plurality of soft sections 70 and a plurality of stiff sections 80. The soft sections 70 have a reduced modulus of elasticity, i.e. they can be stretched in length directions 100 with relatively little force. The stiff sections 80 have an elevated modulus of elasticity, i.e. they require more force to be stretched in length directions 100 by the same distance than the soft sections 70 require. The soft sections 70 all have the same length 130. Also the stiff sections 80 all have the same length 90, which is greater than the length 130 of the soft sections 70. Each stiff section 80 and each soft section 70 extends from a first side edge 200 of the bandage 1 to an opposed second side edge 210.

Arranged in its length direction 100, the bandage 1 comprises a first engagement zone 30 and a second engagement zone 40, both arranged on the first major surface 10 of the bandage 1 and of respective stiff sections 80. Both engagement zones 30, 40, comprise respective engagement means 50, 60 in the form of a plurality of small hooks. The first engagement zone 30 and the second engagement zone 40 are arranged adjacent to opposed longitudinal ends of the leftmost soft section 70a, so that the soft section 70a is arranged, in length direction 100, between the first engagement zone 30 and the second engagement zone 40.

The stiff sections 80 comprise, on their second major surface 20, a third engagement zone 140. The third engagement zone 140 of each stiff section 80 comprises engagement means 160 in the form of a plurality of loops, which are suitably shaped to engage mechanically with hooks that are identical to the hooks of the first engagement zone 30 or of the second engagement zone 40. Once the bandage is applied to a patient's calf, a first portion of the third engagement zone 140 will engage with the first engagement zone 30 of a stiff section 80 different from the stiff section 80 on which the third engagement zone 140 is arranged. A second portion of the third engagement zone 140 will engage with the second engagement zone 40 of a stiff section 80 different from the stiff section 80 on which the third engagement zone 140 is arranged.

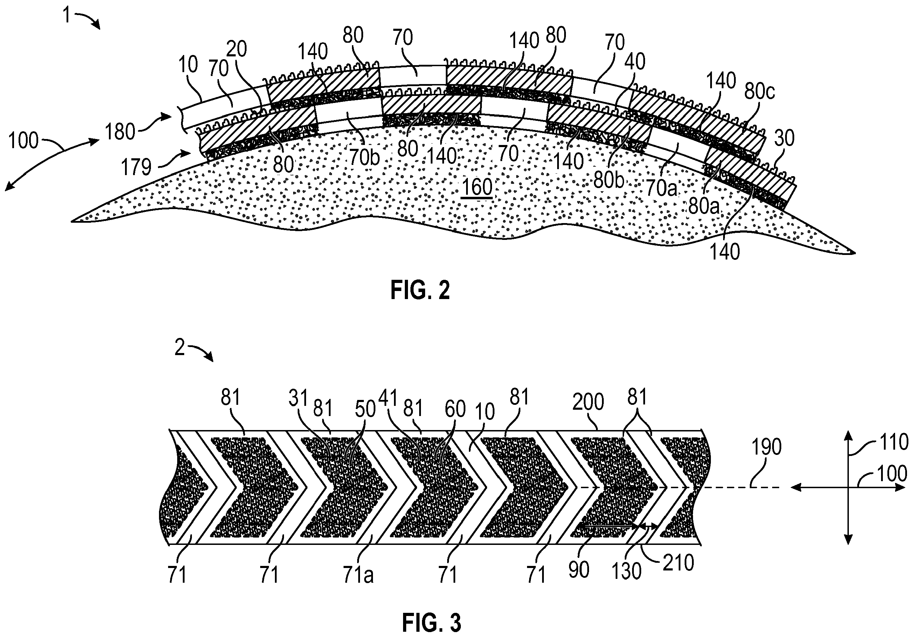

**Figure 2** is a side view of the first compression bandage 1 shown in Figure 1, applied around a calf 160, which is shown in cross section. The bandage 1 is applied around the calf 160 in two turns, resulting in a preceding inner turn 179 and a subsequent outer turn 180 of the same bandage 1. The outer turn 180 is applied over the inner turn 179 with a 50% overlap in width direction, and Figure 2 is a side view of the turns 179, 180 where they overlap. The rightmost stiff sections 80a, 80b of the inner turn 179 comprise, on the first major surface 10 of the bandage 1, respective first and second engagement zones 30, 40 which have mechanical engagement means 50, 60 in the form of hooks. The soft section 70a is arranged between, in length direction 100, the respective first engagement zone 30 and the respective second engagement zone 40.

It can be seen that a right-hand portion of the third engagement zone 140 of the rightmost stiff section 80c of the outer turn 180 is engaged with the first engagement zone 30 on the first major surface 10 of the first stiff section 80a of the preceding, inner turn 179. Also, a left-hand portion of the third engagement zone 140 of the rightmost stiff section 80c of the outer turn 180 is engaged with the second engagement zone 40 on the first major surface 10 of the second stiff section 80b of the preceding, inner turn 179. In this arrangement, the stiff section 80c bridges the soft section 70a.

The stiff sections 80a and 80a are thus connected with each other in two ways: firstly, via the soft zone 70a between them, and secondly via the stiff section 80c of the subsequent turn 180. Because the engagement of the engagement zones 30, 40, 140 prevents relative movement of the stiff sections 80a, 80b, 80c in length directions 100 of the bandage 1, the bandage 1 is considerably stiffer (i.e. resists more to slight increases of the diameter of the calf 160) in the area of the bridged soft section 70a. Before bridging, only the soft section 70a presented a (lower) resistance to diameter increase of the calf 160, while with bridging, the soft section 70a and the bridging stiff section 80c present a considerably higher resistance to such a diameter increase.

The pattern of lengthwise-alternating stiff zones 80 and soft zones 70 of the bandage 1 suggests that engagement means 50, 60 on the first major surface 10 of a stiff section 80 can form a first engagement zone 30 or a second engagement zone 40, depending on if they engage with the first portion or the second portion of the third engagement zone 140 of a stiff section 80 of the subsequent turn 180. This, however, is a question of nomenclature only.

In the left part of Figure 2 one of the soft sections 70, namely the soft section 70b, of the inner turn 179 is not bridged by an overlying stiff section 80 in the upper turn 180. This is due to a mismatch in lengthwise register between the inner turn 179 and the outer turn 180, which itself varies with the circumference from calf to calf. In the area of that unbridged soft section 70b the bandage 1 remains soft, i.e. easy to expand lengthwise, and does not present high resistance against slight increases in diameter of the calf 160. In order to reduce the likelihood of such mismatch in lengthwise register, the pattern of rectangular stiff sections 80 and rectangular soft sections 70 used in the first compression bandage 1 can be replaced with a pattern of chevron-shaped stiff sections and soft sections.

This is illustrated in **Figure 3****,** which shows, in top view, a second compression bandage 2 according to the present disclosure, arranged flat and unexpanded. Similar to the first bandage 1, the second bandage 2 has, arranged sequentially in its length direction 100 in an alternating sequence, a plurality of soft sections 71 and a plurality of stiff sections 81. The stiff sections 81 have a "chevron" shape, symmetric with respect to a central axis 190 of the bandage, all having the same size, the same length 90, and the same orientation, namely pointing towards the right (in Figure 3) along the central axis 190. The soft sections 71 also have a chevron shape, symmetric with respect to the central axis 190, all having the same size, the same length 130, and the same orientation, identical to the orientation of the stiff sections 81. Each stiff section 81 and each soft section 71 extends from a first side edge 200 of the bandage 2 to an opposed second side edge 210.

Arranged in its length direction 100, the second bandage 2 comprises a first engagement zone 31 and a second engagement zone 41, both arranged on the first major surface 10 of the bandage 2 and of respective stiff sections 81. Both engagement zones 31, 41, comprise respective engagement means 50, 60 in the form of a plurality of small hooks. Unlike in the first bandage 1, the engagement zones 31, 41 are smaller than the stiff sections 81 on which they are arranged and do not extend up to the edges of the stiff sections 81. The first engagement zone 31 and the second engagement zone 41 are arranged adjacent to opposed longitudinal ends of the intermediate soft section 71a, so that the intermediate soft section 71a is arranged, in length direction 100, between the first engagement zone 31 and the second engagement zone 41. The soft section 71a and all soft sections 71 are free of hooks and any engagement means.

Except for the chevron-shape of the soft sections 71, of the stiff sections 81, and of the engagement zones 31, 41 arranged on major surfaces of the stiff sections 81, the second bandage 2 is identical to the first bandage 1 of Figures 1 and 2.

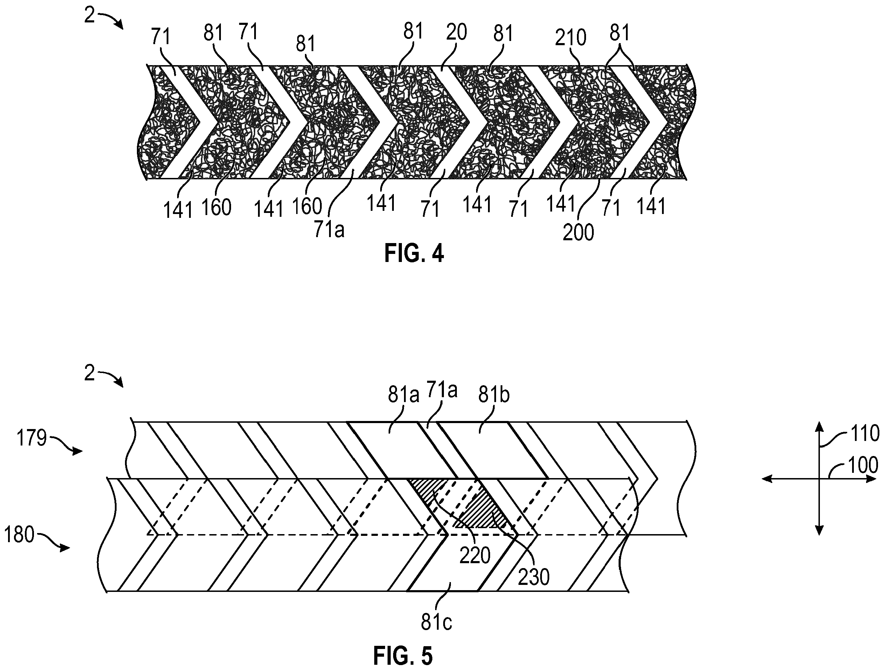

**Figure 4** illustrates, in a bottom view, the second major surface 20 of the second compression bandage 2. The stiff sections 81 comprise, on this second major surface 20, respective third engagement zones 141. Each third engagement zone 141 comprises mechanical engagement means 160 in the form of a plurality of loops, which are suitably shaped to engage mechanically with hooks that are identical to the hooks 50 of the first engagement zone 31 or the second engagement zone 41. The third engagement zones 141 extend from the first side edge 200 to the second opposed side edge 210.
**Figure 5** is a top view of two portions of the second bandage 2, arranged on top of each other, to illustrate how an inner turn 179 and a subsequent outer turn 180 interact, once the bandage 2 is applied around a calf 160 in two circumferential turns with an overlap of about 50% in width direction 110 of the bandage 2. For simplicity, the underlying portion will be referred to as the inner turn 179, the overlying portion as the outer turn 180, reflecting the sequence of turns when the bandage 2 is applied around the calf 160. For greater clarity, the engagement means are not drawn in Figure 5. A first portion 220 of the third engagement zone 141 underneath the stiff section 81c of the outer turn 180 is engaged with the first engagement zone 31 on the first major surface 10 of the first stiff section 81a of the preceding, inner turn 179. Also, a second portion 230 of the third engagement zone 141 on the underside of the stiff section 81c of the outer turn 180 is engaged with the second engagement zone 41 on the first major surface 10 of the second stiff section 81b of the preceding, inner turn 179. In this arrangement, the stiff section 81c bridges the soft section 71a.

It is apparent that other stiff sections 81 of the outer turn 180 bridge other soft sections 71 of the inner turn 179 via the same mechanism, so that the entire bandage 2, after application around the calf, is stiffer. It is also apparent that a subsequent third turn (not shown) will bridge the soft sections 71 of the second turn 180.

Where engagement zones 31, 41, 141 have chevron shapes, a 50% overlap results in the right "arms" of the chevrons of the underlying turn 179 being oriented at an angle with the left "arms" of the chevrons of the overlying turn 180, forming an X pattern. With the angle of the chevron and the length 130 of the soft sections 71 appropriately chosen, this configuration reduces the likelihood of a mismatch in lengthwise register between the inner turn 179 and the outer turn 180 and thus of the occurrence of unbridged soft sections 71.

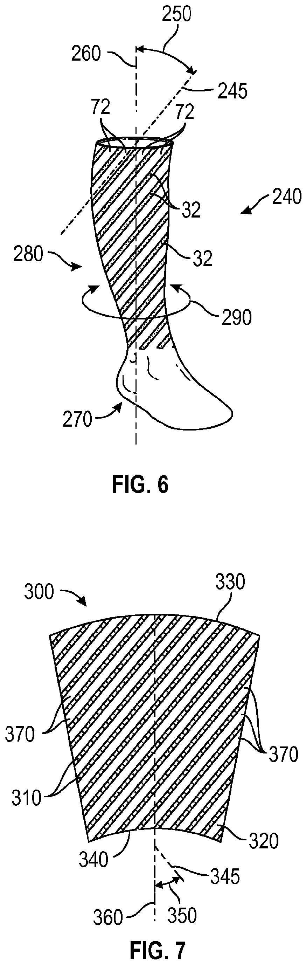

The perspective view of **Figure 6** illustrates how the concept of the present disclosure may be used to provide a compression stocking that is easy to put on, and that is then rendered stiff by applying a stiffening wrap around the stocking.

The compression stocking 240 is shown arranged flat and unexpanded. A foot section 270 is for application over a patient's foot, a calf section 280 is for application over the patient's calf. The stocking 240 is elastic and comprises on its outer major surface and all around its circumference, in an alternating sequence, a plurality of stiff sections carrying engagement zones 32 and a plurality of soft sections 72, arranged between the engagement zones 32. Each of the engagement zones 32 of the stocking 240 comprises respective mechanical engagement means (not shown) on the outer surface, as explained above. The soft sections 72 have a linear, straight elongate shape. They are arranged between the stiff sections carrying engagement zones 32, parallel to each other in a direction 245 forming an angle 250 of about 45° with a longitudinal axis 260 of the stocking 240. The soft sections 72 have a reduced modulus of elasticity in circumferential directions 290. They are thus easily stretchable in circumferential directions 290, so that the stocking 240 readily expands when pulled over the foot and over the calf, and is thus easy to pull on. However, the stocking 240 alone is not stiff enough, i.e. too easily stretchable circumferentially to provide sufficient therapeutic pressure for treatment of leg ulcer.

Once the stocking 240 has been pulled on, a separate element, a so-called stiffening wrap 300 can be applied circumferentially around the calf, over the calf section 280 of the stocking 240. The stiffening wrap 300 is shown, in plan view, as arranged flat and unexpanded in **Figure 7****.** The stiffening wrap 300 is a sheet-like flexible element, made of an expandable material that comprises soft portions having a reduced modulus of elasticity, and stiff portions having an elevated modulus of elasticity, higher than the reduced modulus of elasticity.

The stiffening wrap 300 comprises a linear pattern of a plurality of parallel engagement zones 310 on the one of its major surfaces that, after wrapping around the calf section 280 of the stocking 240, faces towards the patient's skin. These engagement zones are arranged on the stiff portions. The major surface of the stiffening wrap 300 facing towards the patient's skin when in use, referred to as the "inner" major surface 320, is shown in Figure 7. Its upper edge 330 is longer than its lower edge 340, because it is for wrapping around the upper portion of the calf, closer to the knee, where the calf has a larger circumference than it has closer to the foot. The lengths of the edges 330, 340 is chosen such that the stiffening wrap 300 can be wound around a typical calf slightly more than one single time. The height of the stiffening wrap 300, i.e. its extension between the lower edge 340 and the upper edge 330, is chosen such that it fits over the calf portion 280 of the compression stocking 240, between knee and foot of a typical calf. Soft material, i.e. material having a reduced modulus of elasticity in circumferential direction 290 (when the stiffening wrap is arranged around the calf 160) is arranged between stiff sections carrying the engagement zones 310 and forms the soft portions 370, so that soft portions 370 are arranged between adjacent stiff portions carrying engagement zones 310.

The engagement zones 310 of the stiffening wrap 300 comprise engagement means that can engage with the engagement means on the engagement zones 32 on the outer surface of the stocking 240. Such engagement means could be, for example, hook-and-loop systems, where the engagement means on the stocking 240 are loops, and engagement means on the stiffening wrap 300 are hooks that can engage with the loops. Alternative mechanical engagement like mechanical fasteners on the basis of small mushrooms, friction-enhancing layers, magnetic or adhesive engagement means, have been discussed above in the context of compression bandages according to the present disclosure. They can be used on a stocking 240 and a stiffening wrap 300 as described here.

The direction 345 of the straight, linear and parallel engagement zones 310 forms an angle 350 of about 45° with a central axis 360 of the stiffening wrap 300. This central axis 360 passes through the midpoint of the upper edge 330 and the midpoint of the lower edge 340. In use, when the stiffening wrap 300 is wrapped around the stocking 240 and the calf, the central axis 360 is roughly parallel to the longitudinal axis 260 of the stocking 240.

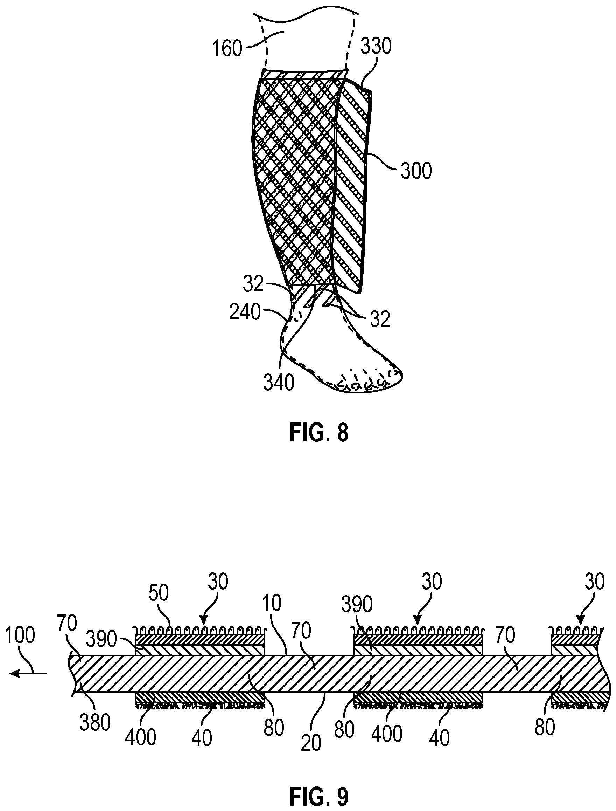

**Figure 8** shows, in a perspective view, a human calf 160 wearing the compression stocking 240 of Figure 6 with the stiffening wrap 300 of Figure 7 wrapped around the calf portion 280 of the stocking 240. Just for enhancing clarity, the stiffening wrap 300 has been drawn as if it were transparent between the engagement zones 310. Thereby it can be seen that, in use, the linear engagement zones 310 of the stiffening wrap 300 and the linear engagement zones 32 of the stocking 240 cross each other and form a crosshatch pattern. This pattern results from the engagement zones 32 of the stocking 240 and the engagement zones of the stiffening wrap 300 forming angles 250, 350 with their respective central axes 260, 360. The crosshatch pattern brings the advantage of a reduced risk of any soft section 72 of the stocking 240 not being bridged by a stiff section 370 of the stiffening wrap 300.

The stiffening wrap 300 is applied with some lengthwise tension over the stocking 240 with its inner surface 320 contacting the outer surface of the stocking 240, whereby the engagement zones 310 of the stiffening wrap engage with the engagement zones 32 of the stocking 240. By this engagement, the stiff sections 370 of the stiffening wrap 300 bridge the soft sections 72 of the stocking 240, so that the soft sections 72 cannot be expanded in circumferential directions 290 easily. The stocking 240 is thus rendered stiff by the application of the stiffening wrap 300. The combination of stocking 240 and stiffening wrap 300 presents a higher resistance to small increases in the diameter of the calf 160, e.g. when the patient walks, which in turn provides for a more effective therapeutic compression of the calf and more effective treatment of leg ulcer.

**Figure 9** is a sectional view of an exemplary flexible material for use in a bandage 1, 2 that is expandable in length direction 100 and comprises soft sections 70, arranged between engagement zones on stiff sections 30. The base is a soft, easily expandable material 380 having a reduced modulus of elasticity. In the area of each stiff section 80, a patch of stiff material 390 is secured to the first major surface 10 of the soft material 380, e.g. adhesively or by welding or stitching. Suitable soft materials and suitable stiff materials are commercially available, e.g. from MITI and other companies mentioned above. The upper surface (in Figure 9) of each stiff section 80 carries an engagement zone 30 which comprises mechanical engagement means in the form of small hook-shaped elements 50.

Likewise, in the area of each stiff section 80, a patch of stiff material 400 is secured to the second major surface 20 of the soft material 380, e.g. adhesively or by welding or stitching. The opposed, lower surface (in Figure 9) of each patch 400 in a stiff section 80 carries a further engagement zone 40 which comprises mechanical engagement means in the form of loops, formed of thin filaments, and suitable to mechanically engage with the hooks of the engagement portions 30 on the upper major surface 10.

The stiffness of the stiff materials 390, 400 renders the portions of the soft material 380 on which the stiff materials 390, 400 are applied stiff and provides them with an elevated modulus of elasticity, turning them into stiff sections 80. The modulus of elasticity of the portions between the stiff sections 80 remains low, so that these sections keep being soft sections 70 after application of the patches of stiff material 390, 400 on the soft material 380.

The result is a material that comprises engagement zones 30, 40 on opposite major surfaces 10, 20, and an alternating sequence of stiff sections 80 and soft sections 70. Such a material can be used to manufacture compression bandages according to the present disclosure.

An alternative way of rendering a portion of an initially soft, easily stretchable material stiff or even rigid may be to coat that portion with a liquid or viscous curable resin, and let the resin penetrate into the material at least to some degree. By curing the resin and thereby hardening it, the resin stiffens the material. A suitable resin is, for example the UV-curable resin described in the U.S. patent application US 2013/01791.

## Claims

1. Elongate elastic compression bandage (1, 2) for application around a human calf (160) in circumferential turns (179, 180) for applying a therapeutic pressure on the calf for treatment of venous leg ulcer,
the bandage having a first major surface (10) and an opposed second major surface (20), wherein the long extension of the bandage, when arranged flat, defines a length direction (100), and
wherein the bandage comprises, arranged in its length direction,
a) a first engagement zone (31), arranged on the first major surface and comprising mechanical engagement means (50),
b) a second engagement zone (41), arranged on the first major surface and comprising mechanical engagement means (60),
c) a soft section (71, 71a),
- arranged, in length direction of the bandage, between the first engagement zone and the second engagement zone,
- having a reduced modulus of elasticity in length direction of less than 0.1 N/cm/% at an elongation of 5% over the unstretched length, when measured on a 5 cm wide specimen on a tensile elongation testing machine at a temperature of 22°C,
d) a stiff section (81)
- having an elevated modulus of elasticity in length direction, higher than the reduced modulus, and of more than 0.03 N/cm/% at an elongation of 5% over the unstretched length, when measured on a 5 cm wide specimen on a tensile elongation testing machine at a temperature of 22°C,
- having a length (90) greater than the length (130) of the soft section, where a length of a section is determined by the longest extension of the section in the length direction of the unexpanded bandage,
- comprising a third engagement zone (141) arranged on the second major surface, comprising mechanical engagement means (160), for engagement with the first engagement zone and with the second engagement zone,
wherein the sections (71, 71a, 81) and the engagement zones (31, 41, 141) are arranged such, that, when applying the bandage around the calf in circumferential turns, a first portion of the third engagement zone can engage, in a subsequent turn, with the first engagement zone, and that a second portion of the third engagement zone can engage, with the second engagement zone, so that the stiff section bridges the soft section;
wherein the plurality of stiff sections being arranged alternating with the plurality of soft sections in length direction of the bandage,
wherein each stiff section of the plurality of stiff sections comprises, on the first major surface (10), a first engagement zone (31) and a second engagement zone (41), and, on the second major surface, a third engagement zone (141), each engagement zone (31, 41, 141) comprising mechanical engagement means (50, 60, 160),
the third engagement zone being arranged such that a first portion of the third engagement zone can engage with the first engagement zone of a first other stiff section of the plurality of stiff sections, and such that a second portion of the third engagement zone can engage with the second engagement zone of a second other stiff section of the plurality of stiff sections, so that the stiff section bridges the soft section between the first other and the second other stiff section; and
wherein the third engagement zones (141) of at least two subsequent stiff sections (81) have congruent "chevron" shapes, oriented parallel to each other and pointing in the same length direction (100) of the bandage, wherein the at least two chevron shapes are spaced from each other in length direction by one or more respective chevron-shaped soft sections.

2. Compression bandage (1, 2) according to claim 1, wherein the bandage extends in width directions (110) between a first side edge (200) and a second side edge (210), and wherein each stiff section and each soft section extends from the first side edge to the second side edge.

3. Compression bandage (1, 2) according to any one of the preceding claims, wherein the bandage extends in width directions between a first side (200) edge and a second side edge (210), wherein each stiff section (81) has a rectangular or a trapezoid shape and extends from the first side edge (200) to the second side edge (210), and wherein each soft section (71, 71a) has a rectangular shape and extends from the first side edge (200) to the second side edge (210).

4. Compression bandage (1, 2) according to any one of the preceding claims, wherein the first and/or the second engagement zone comprises engagement means (50, 60) in the form of a plurality of hooks, and wherein the third engagement zone comprises engagement means (160) in the form of a plurality of loops, shaped suitably to be mechanically engaged with hooks identical to the hooks of the first and/or the second engagement zone.

5. Compression bandage according to any one of the preceding claims, wherein the first and/or the second engagement zone comprises engagement means in the form of a plurality of loops, and wherein the third engagement zone comprises engagement means in the form of a plurality of hooks, shaped suitably to be mechanically engaged with loops identical to the loops of the first and/or the second engagement zone.

6. Compression bandage according to any one of the preceding claims, wherein the first (31) and/or the second (41) engagement zone comprises engagement means (50, 60) in the form of a plurality of mushroom-shaped fastening elements, and wherein the third engagement zone (141) comprises engagement means (160) in the form of a plurality of mushroom-shaped fastening elements, adapted to be mechanically engaged with mushroom-shaped fastening elements identical to the mushroom-shaped fastening elements of the first and/or second engagement zone.

7. Compression bandage according to any one of the preceding claims, wherein the first engagement zone (31) and/or the second engagement zone (41) comprises engagement means (50, 60) in the form of an exposed friction-enhancing layer, adapted to be mechanically engaged with an exposed surface identical to an exposed surface of the third engagement zone (141), or wherein the third engagement zone (141) comprises engagement means (160) in the form of an exposed friction-enhancing layer, adapted to be mechanically engaged with an exposed surface identical to an exposed surface of the first engagement zone (31) or the second engagement zone (41).

8. Compression bandage according to any one of the preceding claims, wherein at least one stiff section (81) comprises apertures for facilitating transmission of air between the second major surface (20) and the first major surface (10).

9. Compression bandage (1, 2) according to any one of the preceding claims, wherein the bandage, when unexpanded and arranged flat, covers a geometric bandage area, and wherein the plurality of stiff sections (81) occupy at least 50% of the geometric bandage area.

10. Compression bandage (2) according to any of the preceding claims, wherein the length (130) of each of the chevron-shaped soft sections (71, 71a) is less than 80% of the smallest length (90) of each of the at least two chevron-shaped stiff sections (81).

## Patentansprüche

1. Längliche elastische Kompressionsbandage (1, 2) zur Aufbringung um eine menschliche Wade (160) in Umfangswindungen (179, 180) zum Ausüben eines therapeutischen Drucks auf die Wade zur Behandlung von venösem Beingeschwür,
wobei die Bandage eine erste Hauptoberfläche (10) und eine gegenüberliegende zweite Hauptoberfläche (20) aufweist, wobei die lange Ausdehnung der Bandage, wenn sie flach angeordnet ist, eine Längsrichtung (100) definiert, und
wobei die Bandage, angeordnet in ihrer Längsrichtung, umfasst
a) eine erste Eingriffszone (31), die auf der ersten Hauptoberfläche angeordnet ist und mechanische Eingriffsmittel (50) umfasst,
b) eine zweite Eingriffszone (41), die auf der ersten Hauptoberfläche angeordnet ist und mechanische Eingriffsmittel (60) umfasst,
c) einen weichen Abschnitt (71, 71a),
- der, in Längsrichtung der Bandage, zwischen der ersten Eingriffszone und der zweiten Eingriffszone angeordnet ist,
- der einen verringerten Elastizitätsmodul in Längsrichtung von weniger als 0,1 N/cm/% bei einer Dehnung von 5 % über die ungedehnte Länge aufweist, wenn an einer 5 cm breiten Probe auf einer Zugdehnungsprüfmaschine bei einer Temperatur von 22 °C gemessen wird,
d) einen steifen Abschnitt (81)
- der einen erhöhten Elastizitätsmodul in Längsrichtung aufweist, der höher als der verringerte Modul ist, und von mehr als 0,03 N/cm/% bei einer Dehnung von 5 % über die ungedehnte Länge, wenn an einer 5 cm breiten Probe auf einer Zugdehnungsprüfmaschine bei einer Temperatur von 22 °C gemessen wird,
- der eine Länge (90) aufweist, die größer ist als die Länge (130) des weichen Abschnitts, wobei eine Länge eines Abschnitts durch die längste Ausdehnung des Abschnitts in Längsrichtung der nicht ausgedehnten Bandage bestimmt ist,
- umfassend eine dritte Eingriffszone (141), die auf der zweiten Hauptoberfläche angeordnet ist, umfassend mechanische Eingriffsmittel (160) zum Eingriff mit der ersten Eingriffszone und mit der zweiten Eingriffszone,
wobei die Abschnitte (71, 71a, 81) und die Eingriffszonen (31, 41, 141) auf derartige Weise angeordnet sind, dass beim Aufbringen der Bandage um die Wade in Umfangswindungen ein erster Abschnitt der dritten Eingriffszone, in einer darauffolgenden Windung, mit der ersten Eingriffszone in Eingriff kommen kann und dass ein zweiter Abschnitt der dritten Eingriffszone mit der zweiten Eingriffszone in Eingriff kommen kann, so dass der steife Abschnitt den weichen Abschnitt überbrückt;
wobei die Mehrzahl von steifen Abschnitten in Längsrichtung der Bandage abwechselnd mit der Mehrzahl von weichen Abschnitten angeordnet ist,
wobei jeder steife Abschnitt der Mehrzahl von steifen Abschnitten auf der ersten Hauptoberfläche (10) eine erste Eingriffszone (31) und eine zweite Eingriffszone (41), und auf der zweiten Hauptoberfläche eine dritte Eingriffszone (141) umfasst, wobei jede Eingriffszone (31, 41, 141) mechanische Eingriffsmittel (50, 60, 160) umfasst,
wobei die dritte Eingriffszone auf derartige Weise angeordnet ist, dass ein erster Abschnitt der dritten Eingriffszone mit der ersten Eingriffszone eines ersten anderen steifen Abschnitts der Mehrzahl von steifen Abschnitten in Eingriff kommen kann, und auf derartige Weise, dass ein zweiter Abschnitt der dritten Eingriffszone mit der zweiten Eingriffszone eines zweiten anderen steifen Abschnitts der Mehrzahl von steifen Abschnitten in Eingriff kommen kann, so dass der steife Abschnitt den weichen Abschnitt zwischen dem ersten anderen und dem zweiten anderen steifen Abschnitt überbrückt; und
wobei die dritten Eingriffszonen (141) von mindestens zwei aufeinanderfolgenden steifen Abschnitten (81) kongruente "Chevron"-Formen aufweisen, die parallel zueinander ausgerichtet sind und in die gleiche Längsrichtung (100) der Bandage zeigen, wobei die mindestens zwei Chevron-Formen in Längsrichtung durch einen oder mehrere jeweilige chevronförmige weiche Abschnitte voneinander beabstandet sind.

2. Kompressionsbandage (1, 2) nach Anspruch 1, wobei sich die Bandage in Breitenrichtungen (110) zwischen einem ersten Seitenrand (200) und einem zweiten Seitenrand (210) erstreckt und wobei sich jeder steife Abschnitt und jeder weiche Abschnitt von dem ersten Seitenrand zu dem zweiten Seitenrand erstreckt.

3. Kompressionsbandage (1, 2) nach einem der vorstehenden Ansprüche, wobei sich die Bandage in Breitenrichtungen zwischen einem ersten Seitenrand (200) und einem zweiten Seitenrand (210) erstreckt, wobei jeder steife Abschnitt (81) eine rechteckige oder trapezförmige Form aufweist und sich von dem ersten Seitenrand (200) zu dem zweiten Seitenrand (210) erstreckt und wobei jeder weiche Abschnitt (71, 71a) eine rechteckige Form aufweist und sich von dem ersten Seitenrand (200) zu dem zweiten Seitenrand (210) erstreckt.

4. Kompressionsbandage (1, 2) nach einem der vorstehenden Ansprüche, wobei die erste und/oder die zweite Eingriffszone Eingriffsmittel (50, 60) in Form einer Mehrzahl von Haken umfasst und wobei die dritte Eingriffszone Eingriffsmittel (160) in Form einer Mehrzahl von Schleifen umfasst, die passend geformt sind, um mechanisch mit Haken in Eingriff gebracht zu werden, die mit den Haken der ersten und/oder der zweiten Eingriffszone identisch sind.

5. Kompressionsbandage nach einem der vorstehenden Ansprüche, wobei die erste und/oder die zweite Eingriffszone Eingriffsmittel in Form einer Mehrzahl von Schleifen umfasst und wobei die dritte Eingriffszone Eingriffsmittel in Form einer Mehrzahl von Haken umfasst, die passend geformt sind, um mechanisch mit Schleifen in Eingriff gebracht zu werden, die mit den Schleifen der ersten und/oder der zweiten Eingriffszone identisch sind.

6. Kompressionsbandage nach einem der vorstehenden Ansprüche, wobei die erste (31) und/oder die zweite (41) Eingriffszone Eingriffsmittel (50, 60) in Form einer Mehrzahl von pilzförmigen Befestigungsmitteln umfasst und wobei die dritte Eingriffszone (141) Eingriffsmittel (160) in Form einer Mehrzahl von pilzförmigen Befestigungsmitteln umfasst, die dazu ausgelegt sind, um mechanisch mit pilzförmigen Befestigungsmitteln in Eingriff gebracht zu werden, die mit den pilzförmigen Befestigungsmitteln der ersten und/oder der zweiten Eingriffszone identisch sind.

7. Kompressionsbandage nach einem der vorstehenden Ansprüche, wobei die erste Eingriffszone (31) und/oder die zweite Eingriffszone (41) Eingriffsmittel (50, 60) in Form einer freiliegenden reibungserhöhenden Schicht umfasst, die dazu ausgelegt sind, mechanisch mit einer freiliegenden Oberfläche in Eingriff gebracht zu werden, die mit einer freiliegenden Oberfläche der dritten Eingriffszone (141) identisch ist, oder wobei die dritte Eingriffszone (141) Eingriffsmittel (160) in Form einer freiliegenden reibungserhöhenden Schicht umfasst, die dazu ausgelegt sind, mechanisch mit einer freiliegenden Oberfläche in Eingriff gebracht zu werden, die mit einer freiliegenden Oberfläche der ersten Eingriffszone (31) oder der zweiten Eingriffszone (41) identisch ist.

8. Kompressionsbandage nach einem der vorstehenden Ansprüche, wobei mindestens ein steifer Abschnitt (81) Öffnungen zum Erleichtern der Übertragung von Luft zwischen der zweiten Hauptoberfläche (20) und der ersten Hauptoberfläche (10) umfasst.

9. Kompressionsbandage (1, 2) nach einem der vorstehenden Ansprüche, wobei die Bandage, wenn sie nicht ausgedehnt und flach angeordnet ist, eine geometrische Bandagenfläche bedeckt, und wobei die Mehrzahl von steifen Abschnitten (81) mindestens 50 % der geometrischen Bandagenfläche einnimmt.

10. Kompressionsbandage (2) nach einem der vorstehenden Ansprüche, wobei die Länge (130) jedes der chevronförmigen weichen Abschnitte (71, 71a) weniger als 80 % der kleinsten Länge (90) von jedem der mindestens zwei chevronförmigen steifen Abschnitte (81) beträgt.

## Revendications

1. Bandage élastique de compression allongé (1, 2) pour son application autour d'un mollet humain (160) en tours circonférentiels (179, 180) pour l'application d'une pression thérapeutique sur le mollet pour le traitement d'un ulcère de jambe veineux,
le bandage ayant une première surface principale (10) et une deuxième surface principale opposée (20), dans lequel l'extension longue du bandage, lorsqu'elle est disposée à plat, définit un sens de la longueur (100), et
dans lequel le bandage comprend, disposé dans le sens de sa longueur,
a) une première zone de mise en prise (31), disposée sur la première surface principale et comprenant des moyens de mise en prise mécaniques (50),
b) une deuxième zone de mise en prise (41), disposée sur la première surface principale et comprenant des moyens de mise en prise mécaniques (60),
c) une section souple (71, 71a),
- disposée, dans le sens de la longueur du bandage, entre la première zone de mise en prise et la deuxième zone de mise en prise,
- ayant un module d'élasticité réduit dans le sens de la longueur inférieur à 0,1 N/cm/% à un allongement de 5 % sur la longueur non étirée, lorsqu'il est mesuré sur un échantillon de 5 cm de large sur une machine de test d'allongement à la traction à une température de 22 °C,
d) une section rigide (81)
- ayant un module d'élasticité élevé dans le sens de la longueur, plus important que le module réduit, et supérieur à 0,03 N/cm/% à un allongement de 5 % sur la longueur non étirée, lorsqu'il est mesuré sur un échantillon de 5 cm de large sur une machine de test d'allongement à la traction à une température de 22 °C,
- ayant une longueur (90) supérieure à la longueur (130) de la section souple, où une longueur d'une section est déterminée par l'extension la plus longue de la section dans le sens de la longueur du bandage non expansé,
- comprenant une troisième zone de mise en prise (141) disposée sur la deuxième surface principale, comprenant des moyens de mise en prise mécaniques (160), pour la mise en prise avec la première zone de mise en prise et avec la deuxième zone de mise en prise,
dans lequel les sections (71, 71a, 81) et les zones de mise en prise (31, 41, 141) sont disposées de telle sorte, que, lors de l'application du bandage autour du mollet en tours circonférentiels, une première partie de la troisième zone de mise en prise peut venir en prise, en un tour ultérieur, avec la première zone de mise en prise, et qu'une deuxième partie de la troisième zone de mise en prise peut venir en prise, avec la deuxième zone de mise en prise, de sorte que la section rigide ponte la section souple ;
dans lequel la pluralité de sections rigides étant disposées en alternance avec la pluralité de sections souples dans le sens de la longueur du bandage,
dans lequel chaque section rigide de la pluralité de sections rigides comprend, sur la première surface principale (10), une première zone de mise en prise (31) et une deuxième zone de mise en prise (41), et, sur la deuxième surface principale, une troisième zone de mise en prise (141), chaque zone de mise en prise (31, 41, 141) comprenant des moyens de mise en prise mécaniques (50, 60, 160),
la troisième zone de mise en prise étant disposée de telle sorte qu'une première partie de la troisième zone de mise en prise peut venir en prise avec la première zone de mise en prise d'une première autre section rigide de la pluralité de sections rigides, et de telle sorte qu'une deuxième partie de la troisième zone de mise en prise peut venir en prise avec la deuxième zone de mise en prise d'une deuxième autre section rigide de la pluralité de sections rigides, de sorte que la section rigide ponte la section souple entre la première autre et la deuxième autre section rigide ; et
dans lequel les troisièmes zones de mise en prise (141) d'au moins deux sections rigides ultérieures (81) ont des formes congruentes en « chevron », orientées parallèlement les unes aux autres et pointant dans le même sens de la longueur (100) du bandage, dans lequel les au moins deux formes en chevron sont espacées les unes des autres dans le sens de la longueur par une ou plusieurs sections souples en forme de chevron respectives.

2. Bandage de compression (1, 2) selon la revendication 1, dans lequel le bandage s'étend dans des sens de la largeur (110) entre un premier bord latéral (200) et un deuxième bord latéral (210), et dans lequel chaque section rigide et chaque section souple s'étendent du premier bord latéral au deuxième bord latéral.

3. Bandage de compression (1, 2) selon l'une quelconque des revendications précédentes, dans lequel le bandage s'étend dans des sens de la largeur entre un premier bord latéral (200) et un deuxième bord latéral (210), dans lequel chaque section rigide (81) a une forme rectangulaire ou trapézoïdale et s'étend du premier bord latéral (200) au deuxième bord latéral (210), et dans lequel chaque section souple (71, 71a) a une forme rectangulaire et s'étend du premier bord latéral (200) au deuxième bord latéral (210).

4. Bandage de compression (1, 2) selon l'une quelconque des revendications précédentes, dans lequel la première et/ou la deuxième zone de mise en prise comprend des moyens de mise en prise (50, 60) sous la forme d'une pluralité de crochets, et dans lequel la troisième zone de mise en prise comprend des moyens de mise en prise (160) sous la forme d'une pluralité de boucles, formées de manière appropriée pour venir en prise mécaniquement avec des crochets identiques aux crochets de la première et/ou la deuxième zone de mise en prise.

5. Bandage à compression selon l'une quelconque des revendications précédentes, dans lequel la première et/ou la deuxième zone de mise en prise comprend des moyens de mise en prise sous la forme d'une pluralité de boucles, et dans lequel la troisième zone de mise en prise comprend des moyens de mise en prise sous la forme d'une pluralité de crochets, formés de manière appropriée pour venir en prise mécaniquement avec des boucles identiques aux crochets de la première et/ou la deuxième zone de mise en prise.

6. Bandage de compression selon l'une quelconque des revendications précédentes, dans lequel la première (31) et/ou la deuxième (41) zone de mise en prise comprend des moyens de mise en prise (50, 60) sous la forme d'une pluralité d'éléments de fixation en forme de champignon, et dans lequel la troisième zone de mise en prise (141) comprend des moyens de mise en prise (160) sous la forme d'une pluralité d'éléments de fixation en forme de champignon, adaptés pour venir en prise mécaniquement avec des éléments de fixation en forme de champignon identiques aux éléments de fixation en forme de champignon de la première et/ou la deuxième zone de mise en prise.

7. Bandage de compression selon l'une quelconque des revendications précédentes, dans lequel la première zone de mise en prise (31) et/ou la deuxième zone de mise en prise (41) comprend des moyens de mise en prise (50, 60) sous la forme d'une couche d'amélioration de frottement exposée, adaptée pour venir en prise mécaniquement avec une surface exposée identique à une surface exposée de la troisième zone de mise en prise (141), ou dans lequel la troisième zone de mise en prise (141) comprend des moyens de mise en prise (160) sous la forme d'une couche d'amélioration de frottement exposée, adaptée pour venir en prise mécaniquement avec une surface exposée identique à une surface exposée de la première zone de mise en prise (31) ou la deuxième zone de mise en prise (41).

8. Bandage de compression selon l'une quelconque des revendications précédentes, dans lequel au moins une section rigide (81) comprend des ouvertures pour faciliter la transmission de l'air entre la deuxième surface principale (20) et la première surface principale (10).

9. Bandage de compression (1, 2) selon l'une quelconque des revendications précédentes, dans lequel le bandage, lorsqu'il est non expansé et disposé à plat, recouvre une aire géométrique de bandage, et dans lequel la pluralité de sections rigides (81) occupent au moins 50 % de l'aire géométrique de bandage.

10. Bandage de compression (2) selon l'une quelconque des revendications précédentes, dans lequel la longueur (130) de chacune des sections souples en forme de chevron (71, 71a) est inférieure à 80 % de la plus petite longueur (90) de chacune des au moins deux sections rigides en forme de chevron (81).
